# EUROPEAN PATENT APPLICATION

(11) **EP 4 431 088 A1**
(43) Date of publication of application: **18.09.2024**
(21) Application number: 24382217.8
(22) Date of filing: 28.02.2024
(51) Int. Cl.: A61K 9/20, A61K 31/155, A61K 31/70, A61P 3/10, A61P 9/04, A61P 13/12

(54) **PHARMACEUTICAL COMPOSITIONS COMPRISING DAPAGLIFLOZIN AND METFORMIN**

(30) Priority: 06.03.2023 EP 23382210; 06.03.2023 EP 23382209
(71) Applicant: Galenicum Health S.L.U., 08950 Esplugues de Llobregat (ES)
(72) Inventor: Gómez Coello, Luis, Esplugues de Llobregat (ES); Rubio Pérez-Seva, María, Esplugues de Llobregat (ES)
(74) Representative: Torrejón-Nieto, Javier

(57) **Abstract**

The present invention relates to a uniform pharmaceutical composition comprising dapagliflozin, metformin and at least one surfactant. Manufacturing processes of such composition are also described.

## Description

### FIELD

The present invention relates to a uniform pharmaceutical composition comprising dapagliflozin, metformin and at least one surfactant. Manufacturing processes of such composition are also described.

### STATE OF THE ART

Diabetes mellitus type 2 is a chronic and progressive disease arising from a complex pathophysiology involving the dual endocrine defects of insulin resistance and impaired insulin secretion. The treatment of diabetes mellitus type 2 typically begins with diet and exercise, followed by oral antidiabetic monotherapy. For many patients, these regimens do not sufficiently control glycemia during long-term treatment, leading to a requirement for combination therapy within several years following diagnosis.

Dapagliflozin, also known as (1S)-1,5-anhydro-1-C-[4-chloro-3-[(4-ethoxyphenyl) methyl]phenyl]-D-glucitol or (2S,3R,4R,5S,6R)-2-(3-(4-etoxybenzyl)-4-chlorophenyl)-6-hydroxy methyltetrahydro-2H-pyran-3,4,5-triol, is a sodium-glucose cotransporter 2 (SGLT-2) inhibitor. This cotransporter is responsible for the reabsorption of most of the glucose from the lumen of the renal tubule. By inhibiting SGLT2, dapagliflozin reduces the reabsorption of the filtered glucose and lowers the renal threshold for glucose. This improves urinary glucose excretion and blood glucose control, which is particularly efficient for the treatment of diabetes mellitus type 2. Dapagliflozin has the following structure (compound I):

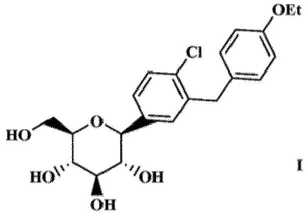

Dapagliflozin is disclosed in EP 1 506 211 B. Further, EP 2 069 374 B discloses dapagliflozin in the form of crystalline SC-3 (S)-propylene glycol hydrate ((S)-PG (SC-3)) and methods for preparing it.

Another compound that has been widely used for the treatment of diabetes mellitus type 2 is metformin. Metformin hydrochloride is a 1,1-dimethylbiguanide hydrochloride with the following structure (II).

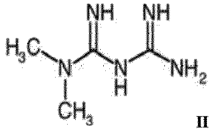

Dapagliflozin and metformin are sold together in a fixed dose combination to improve glycemic control in adults with diabetes mellitus type 2 under the brand name Xigduo^{®} and Xigduo XRO. The commercial strengths are respectively 5 mg/850 mg and 5 mg/1000mg for Xigduo^{®} and 5 mg/500 mg, 5 mg/1000mg, 10 mg/500 mg, 10 mg/1000 mg and 2.5 mg/1000mg for Xigduo XR^{®}.

A fixed dose composition comprising dapagliflozin and metformin presents several challenges. Firstly, the large drug-to-drug ratio between metformin and dapagliflozin is a critical issue vis a vis dapagliflozin content uniformity. Dapagliflozin is present in a relatively small amount and it is difficult to have a uniform distribution that would avoid variation in content. Additionally, dapagliflozin and metformin present very different physical properties. Specifically, metformin has a very poor compaction which makes it difficult to produce tablets having acceptable mechanical strength.

EP 2 498 759 B discloses dapagliflozin-metformin tablets with a good content uniformity and tensile strength by using hydroxypropyl cellulose (HPC) as binder.

EP 4 008 316 A discloses dapagliflozin-metformin tablets with a good content uniformity by specifically using sodium croscarmellose as disintegrant.

WO2021176096A discloses dapagliflozin-metformin tablets with a good content uniformity by using HPC as binder. Specifically, homogeneous granules are obtained by using a binder solution of an organic solvent such as ethanol.

However, there is still a need for an effective dapagliflozin-metformin composition having a good content uniformity, compressibility and tablet hardness, with desired disintegration and dissolution profiles.

### SUMMARY OF THE INVENTION

The first aspect of the present invention relates to a pharmaceutical composition comprising dapagliflozin, metformin and at least one surfactant.

A second aspect of the present invention deals with a process for the preparation of the said pharmaceutical composition.

A third aspect of the present invention provides said pharmaceutical composition for use as a medicament, preferably for the treatment of diabetes mellitus type 2, symptomatic chronic heart failure with reduced ejection fraction and chronic kidney disease, more preferably for use in diabetes mellitus type 2.

### DESCRIPTION OF THE INVENTION

The present invention provides a stable, uniform dapagliflozin-metformin composition comprising at least one surfactant. The present invention also provides a method of manufacturing which is cost-effective, simple and easy for industrial production presenting good technological properties (flowability, compaction, hardness, disintegration, dissolution, uniformity and stability).

Surprisingly, the dapagliflozin-metformin composition of the following invention has excellent uniformity, stability, solubility, flowability, compaction, hardness, friability, disintegration and dissolution profiles. The composition developed is simple and does not require the use of complicated preparation procedures as those disclosed in the art for formulating dapagliflozin and metformin.

### Definitions

As used in the present disclosure, the following words, phrases, and symbols are generally intended to have the meanings as set forth below, except to the extent that the context in which they are used indicates otherwise.

All percentages are expressed by weight (w/w) unless specifically noted otherwise.

Unless otherwise indicated, all the analysis methods are carried out according to the European Pharmacopoeia.

Disclosed compositions of the present invention comprise dapagliflozin. As used herein, the term "dapagliflozin" refers to the free base or any pharmaceutically acceptable salt, solvate or hydrate thereof. Preferably, dapagliflozin is in the form of (S)-propylene glycol solvate ((S)-PG) or also known as (S)-propanediol solvate ((S)-PG). More preferably, dapagliflozin (S)-propylene glycol solvate is in the form of (S)-propylene glycol monohydrate.

Dapagliflozin or any pharmaceutically acceptable salt, solvate or hydrate can be in a crystalline or non-crystalline solid form. Preferably, dapagliflozin is crystalline. More preferably dapagliflozin is in the form of (S)-propylene glycol solvate monohydrate crystalline form SC-3 ((S)-PG-(SC-3)).

Disclosed compositions of the present invention comprise metformin. As used herein, the term "metformin" refers to the free base or any pharmaceutically acceptable salt thereof. Preferably, metformin is in the form of hydrochloride.

Preferably, disclosed compositions of the present invention are free of sitagliptin or any physiologically acceptable salts thereof.

As used herein, the term "uniform" refers to the fact that the content of the active ingredient in the tablets of a pharmaceutical batch has to be homogeneous (Ph.Eur. 2.9.40 & USP 905). According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published October 2003).

The term "stable" as used herein refers to the pharmaceutical composition according to the invention wherein the total content of impurities originated from the decomposition of the active ingredient does not exceed 5 % area, preferably 3 % area, more preferably 2 % area and most preferably 1 % area determined by liquid chromatography (HPLC) if such a composition is stored for at least 1 month at 40°C and 75 % relative humidity (RH).

As used herein, the term "solid oral dosage form" refers to an oral pharmaceutical composition in the form of a tablet, capsule, sachet, granules, powders, pellets, beads, cores, etc. Preferably, the solid oral dosage form is free of effervescent components. By effervescent components it is understood those components that are designed to dissolve in water and release carbon dioxide. Examples of such components comprise a base such as carbonate, sesquicarbonate or bicarbonate salts.

As used herein, the term "immediate release" refers to a pharmaceutical composition having a dissolution performance such as 60% or more of the active ingredient within 60 minutes. Preferably, the immediate release composition as herein disclosed releases at least 80 % of the active ingredient in 60 minutes. More preferably, the pharmaceutical composition as herein disclosed releases at least 80 % of the active ingredient in 45 min, even more preferably in 35 min and more preferably in 30 min.

As used herein, "surfactant" refers to pharmaceutically acceptable excipient or excipients which increase the hydrophilicity of the dosage form.

The term "filler" as used herein refers to pharmaceutically acceptable excipient or excipients added to the bulk volume of the active ingredient making up the solid composition. As a result, the size of the solid composition increases, which makes its size suitable for handling.

As used herein, "disintegrant" means a substance or a mixture of substances added to a tablet to facilitate its breakup or disintegration after administration.

As used herein, "lubricant" means a substance or a mixture of substances that reduce friction between the composition and the surfaces of the apparatus used to compact the composition into a compressed form.

As used herein, "glidant" refers to a substance or substances that improve the flowability of the pharmaceutical compositions when they are in the form of a powder.

As used herein, "binder" refers to a substance that imparts cohesive properties to a formulation. Binders ensure that tablets, powders, granules and others can be formed by the required mechanical strength.

As used herein, "solvent" refers to any liquid substance or substances in which the active ingredient can be dissolved.

As used herein, "batch" refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

As used herein, "particle size" means the cumulative volume size distribution as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

### FIRST ASPECT

In a first aspect, the present invention relates to a pharmaceutical composition comprising dapagliflozin, metformin and at least one surfactant. Preferably, dapagliflozin is in the form of dapagliflozin (S) propylene glycol monohydrate and metformin is in the form of hydrochloride salt. More preferably, dapagliflozin (S) propylene glycol monohydrate is in the crystalline form. Even more preferably, dapagliflozin (S) propylene glycol monohydrate is in the crystalline form SC-3.

In another embodiment of the present invention, the at least one surfactant is selected from polyoxyethylene sorbitan fatty acid ester, polysorbates (for example polysorbate 20, polysorbate 40, polysorbate 60, polysorbate 65, polysorbate 80, polysorbate 85, micro-encapsulated polysorbate 80 such as SEPITRAPTM 80, micro-encapsulated polyoxyl 40 hydrogenated castor oil such as SEPITRAPTM 4000), polyoxyethylene castor oil derivatives polyoxyethylene hydrogenated castor oil (for example CREMOPHOR), alkyl poly(ethylene oxide), copolymers of poly(ethylene oxide) and poly(proplylene oxide) commercially called as poloxamers or poloxamines, polyoxyethylene alkyl ethers, polyoxyethylene alkylaryl ethers, polyethylene glycol fatty acid ester, polyethylene alkyl ether, polyethylene stearate, alkylene glycol fatty acid mono ester, polyoxyethylene stearates, sucrose esters of fatty acids, PEG-8 Caprylic-Capric glycerides (DUBCARE GPE 810), saturated polyglycolized glycerides, ethoxylated hydrogenated castor oil, phosphatidyl choline, phospholipids, medium chain triglycerides, docusate sodium, lecithin, sorbitan monostearate (SPAN 860), sorbitan monopalmitate (SPAN 40), sorbitan monolaurate (SPAN 20 or SPAN 80), ascorbyl palmitate, sodium lauryl sulfate, sodium dodecyl sulfate, sodium lauryl sulfoacetate, ammonium lauryl sulfate, benzalkonium chloride, tocopherol, tocopherol PEG succinate, and mixtures thereof. Preferably, the at least one surfactant is selected from sodium lauryl sulphate, polysorbates (for example polysorbate 20, polysorbate 40, polysorbate 60, polysorbate 65, polysorbate 80, polysorbate 85, micro-encapsulated polysorbate 80 such as SEPITRAPTM 80, micro-encapsulated polyoxyl 40 hydrogenated castor oil such as SEPITRAPTM 4000), ethoxylated hydrogenated castor oil, sorbitan monolaurate (SPAN 20 or SPAN 80), benzalkonium chloride and mixtures thereof. More preferably, the at least one surfactant is selected from sodium lauryl sulphate, polysorbates (for example polysorbate 20, polysorbate 40, polysorbate 60, polysorbate 65, polysorbate 80, polysorbate 85, micro-encapsulated polysorbate 80 such as SEPITRAPTM 80, micro-encapsulated polyoxyl 40 hydrogenated castor oil such as SEPITRAPTM 4000) and mixtures thereof. Even more preferably, the at least one surfactant is selected from sodium lauryl sulphate.

Surfactants are frequently used from 0.001 wt% to 10 wt% by weight of the formulation, preferably from 0.001 wt% to 1 wt%.

The inventors have found that the addition of a surfactant allows the obtaining of the stable, uniform and homogeneous pharmaceutical compositions of the invention with desirable dissolution profiles.

In another embodiment of the present invention, the pharmaceutical composition comprises an amount of dapagliflozin per unit dose equivalent to 2.5 mg, 5 mg or 10 mg of the active ingredient as free base. Preferably, the pharmaceutical composition comprises an amount of dapagliflozin per unit dose equivalent to 5 mg.

In another embodiment of the present invention, the pharmaceutical composition comprises an amount of metformin per unit dose equivalent to 500 mg, 850 mg or 1000 mg of the active ingredient as free base. Preferably, the pharmaceutical composition comprises an amount of metformin per unit dose equivalent to 850 mg or 1000 mg.

In another embodiment of the present invention the pharmaceutical composition is in the form of an immediate release dosage form. Preferably, in the form of a solid oral dosage form.

In another embodiment of the present invention, the pharmaceutical composition is a tablet or a capsule. Preferably the pharmaceutical composition is a tablet. Even more preferably the tablet is free of effervescent components.

In another embodiment of the present invention, the pharmaceutical composition further comprises at least one filler, disintegrant, lubricant, glidant, solvent, binder and mixtures thereof.

In another preferred embodiment of the first aspect of the invention, the pharmaceutical composition is free of binder.

In another embodiment of the present invention, the pharmaceutical composition further comprises at least one filler, disintegrant, lubricant, glidant, solvent and mixtures thereof.

In a preferred embodiment of the first aspect of the present invention, the said pharmaceutical composition further comprises at least one filler. Examples of fillers are lactose (monohydrate, spray-dried monohydrate, anhydrous and the like), mannitol, xylitol, dextrose, sorbitol, compressible sugar, microcrystalline cellulose, starch, pregelatinized starch, dextrates, dextran, dextrin, dextrose, maltodextrin, fructose, kaolin, mannitol, lactitol, maltose, isomaltose, xylitol, inositol, starch, calcium carbonate, calcium phosphate, dibasic calcium phosphate, tribasic calcium phosphate, calcium sulfate, sodium alginate, magnesium carbonate, magnesium oxide and mixtures thereof. Different types of microcrystalline cellulose may be suitable for use in the formulations described herein. Examples of microcrystalline cellulose include Avicel^{®} types: PH101, PH102, PH103, PH105, PH 112, PH113, PH200, PH301, and other types of microcrystalline cellulose, such as silicified microcrystalline cellulose (SMCC). Preferably, the at least one filler is selected from calcium phosphate dibasic, calcium phosphate tribasic, lactose, lactose monohydrate, anhydrous lactose, mannitol, sorbitol, magnesium carbonate, maltose, isomaltose, xylitol, starch and mixtures thereof. More preferably, the at least one filler is selected from isomaltose, calcium phosphate dibasic, calcium phosphate tribasic, mannitol and mixtures thereof. Even more preferably, the at least one filler is selected from calcium phosphate dibasic or tribasic.

Fillers are frequently used in an amount ranging from 10 wt% to 75 wt% weight of the formulation.

In a preferred embodiment of the first aspect of the present invention, the said pharmaceutical composition further comprises at least one disintegrant. Examples of disintegrants include sodium starch glycolate, sodium carboxymethyl cellulose, calcium carboxymethyl cellulose, sodium croscarmellose, crospovidone, polyvinylpyrrolidone, methylcellulose, microcrystalline cellulose, starch, pregelatinized starch, starch and sodium alginate. Preferably, the at least one disintegrant is selected from croscarmellose, sodium croscarmellose, crospovidone, starch, sodium starch glycolate, carmellose and mixtures thereof. More preferably, the at least one disintegrant is selected from crospovidone, sodium croscarmellose, sodium starch glycolate and mixtures thereof. Even more preferably, the at least one disintegrant is selected from sodium starch glycolate.

Disintegrants are frequently used in an amount ranging from 0.1 wt% to 25 wt% by weight of the formulation, preferably from 0.3 wt% to 20 wt%.

In a preferred embodiment, the pharmaceutical composition comprises at least one lubricant. Examples of lubricants include magnesium stearate, calcium stearate, zinc stearate, sodium stearyl fumarate, stearyl alcohol, cetostearyl alcohol, talc, stearic acid, waxes, light mineral oil, glyceryl behenate, glyceryl palmitostearate, glyceryl monostearate, glyceryl monooleate, hydrogenated vegetable oils, polyethylene glycols, alkyl sulfates, or sodium benzoate and mixtures thereof. Preferably, the at least one lubricant is selected from magnesium stearate, sodium stearyl fumarate and stearic acid. More preferably, the at least one lubricant is selected from stearic acid, sodium stearyl fumarate or mixtures thereof.

Lubricants are frequently used from 0.25 wt% to 10 wt% by weight of the formulation.

In a preferred embodiment, the pharmaceutical composition comprises at least one glidant. Examples of glidants include silicon dioxide, colloidal silicon dioxide, magnesium oxide, magnesium silicate, magnesium trisilicate, talc, and other forms of silicon dioxide, such as aggregated silicates and hydrated silica. Preferably, the at least one glidant is selected from colloidal silicon dioxide.

Glidants are frequently used from 0.25 wt% to 10 wt% by weight of the formulation.

In a preferred embodiment, the pharmaceutical composition comprises at least one binder. Examples of binders suitable for use in the present invention include: hydroxypropyl methyl cellulose, hydroxypropyl cellulose, hydroxyethyl cellulose, ethyl cellulose, polyvinyl alcohol, polyethylene glycol, waxes, povidone, copovidone, crospovidone or mixtures thereof. More preferably, the at least one binder is selected from povidone, copovidone or mixtures thereof. Even more preferably the at least one binder is selected from povidone.

Binders are frequently used from 0 wt% to 15 wt% by weight of the formulation.

In another preferred embodiment of the first aspect of the invention, the pharmaceutical composition is free of binders.

In a preferred embodiment, the pharmaceutical composition comprises at least one solvent. Examples of solvents include water, ethanol, methanol, acetone, isopropanol, ethyl acetate, acetonitrile, n-hexane, and cyclohexane or mixtures thereof. Preferably the at least one solvent is water and is free of any organic solvent.

In another preferred embodiment of the first aspect of the invention, the pharmaceutical composition is free of organic solvents.

In another preferred embodiment of the first aspect of the invention, dapagliflozin or any pharmaceutically acceptable salt, solvate or hydrate thereof is micronized. In a preferred embodiment, dapagliflozin or any pharmaceutically acceptable salt, solvate or hydrate thereof has a particle size volume distribution with a D90 less than 200 µm, preferably less than 150 µm, more preferably from less than 100 µm. The particle size volume distribution is analysed by laser diffraction spectroscopy using a Malvern Mastersizer 3000 particle size analyzer.

In a preferred embodiment, the composition is film coated with a coating agent, preferably comprising one or more pharmaceutically acceptable polymers, optionally one or more pharmaceutically acceptable plasticizers, and optionally one or more pharmaceutically acceptable pigments.

More preferably, the coating agent is selected from hypromellose, macrogol 400, polysorbate 80 and titanium dioxide (commercially available as Opadry^{®} White), or hypromellose, macrogol 400, titanium dioxide, iron oxide yellow and iron oxide red (commercially available as Opadry^{®} Brown), or hypromellose, macrogol 400, titanium dioxide, iron oxide red, iron oxide black (commercially available as Opadry^{®} Brown) or polyvinyl alcohol, titanium dioxide, macrogol, talc, iron oxide yellow, iron oxide red (commercially available as Opadry^{®} Orange), or polyvinyl alcohol, titanium dioxide, macrogol, talc, iron oxide yellow, iron oxide black (commercially available as Opadry^{®} Yellow) and mixtures thereof.

The one or more polymer agents of the pharmaceutically acceptable coating agent are preferably selected from one or more of hydroxypropyl cellulose, hydroxypropylmethyl cellulose, ethyl cellulose, polyvinyl alcohol, polyethylene glycol, polyethylene glycol-polyvinyl alcohol graftpolymer, polysorbate and mixtures thereof.

The one or more pigments are preferably selected from the group of titanium dioxide, aluminium lakes, iron oxides and mixtures thereof.

Such coating agents may optionally further comprise one or more additional adjuvants such as a detackifier, flow aid, surfactant, and secondary film-forming polymer. Examples of optional detackifiers include lecithin, stearic acid, mineral oil, modified derivatized starch, tapioca dextrin, polyethylene glycol and mixtures thereof. Examples of optional secondary film-forming polymers include sodium alginate, propylene glycol alginate, and polyvinylpyrrolidone. Examples of optional surfactants include dioctyl sodium sulfosuccinate, polysorbate 80 and mixtures thereof. Examples of optional flow aids include talc, fumed silica, bentonite, hydrogenated vegetable oils, stearines, waxes and mixtures thereof. In a preferred embodiment the coating is free of surfactant.

In another aspect, the present invention relates to a pharmaceutical batch comprising at least 20,000 units, preferably at least 50,000 units, more preferable at least 100,000 units of the pharmaceutical composition of the first aspect. In a preferred embodiment, the content of the active ingredient is uniform. In a preferred embodiment, the pharmaceutical compositions are packaged in a blister pack of aluminum/PVC, aluminum/aluminum or PVC/PE/PVDC/aluminum.

For the release of a pharmaceutical batch the distribution of the active ingredient in the tablets has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5200.14). It is therefore essential to assure that the test batch is uniform.

Each of the embodiments of the present invention described herein may be combined with one or more other embodiments of the present invention described herein that are not inconsistent with the embodiment(s) with which it is combined.

### SECOND ASPECT

In a second aspect, the present invention relates to a process for the manufacture of a pharmaceutical composition of the first aspect.

Pharmaceutical compositions of the present invention may be manufactured using conventional manufacturing techniques. Such conventional manufacturing techniques are, for instance, mixing, dissolving, granulating, dragee-making, levigating, emulsifying, encapsulating, entrapping or compression processes. Specifically, the composition of the present invention may be prepared, using standard techniques and manufacturing processes well known in the art, such as direct compression, wet granulation or dry granulation, hot melt granulation, hot melt extrusion, fluidized bed granulation, extrusion/spheronization, slugging, spray drying and solvent evaporation.

One conventional manufacturing technique for the preparation of the formulation of the present invention is compression.

Another conventional manufacturing technique for the preparation of the formulation of the present invention is granulation, either dry or wet granulation, preferably wet granulation.

The term "granulation" includes dry and wet granulation techniques. The term "wet granulation" refers to any process comprising the steps of addition of a liquid to the powder starting materials, preferably kneading, and drying to yield a solid dosage form. The term "dry granulation" refers to any process that comprises compacting the powder, usually either by slugging or with a roller compactor, and preferably milling the compacted powder to obtain granules. No liquid is employed for the dry granulation.

In order to obtain the said granules intragranular excipients can be used. Intragranular excipients are selected from surfactants, fillers, disintegrants, lubricants, glidants, solvents, binders and mixtures thereof. Preferably, intragranular excipients are selected from surfactants, solvents and mixtures thereof.

Once the granules are obtained, they can be compressed, optionally with other pharmaceutical excipients, extragranular excipients, into tablets. Alternatively, the granules can be filled into capsules.

The extragranular excipients which are compressed together with the granules can be selected from surfactants, fillers, disintegrants, lubricants, glidants, solvents and mixtures thereof. Preferably, extragranular excipients are selected from fillers, disintegrants, lubricants glidants and mixtures thereof.

In an embodiment of the second aspect, the present invention relates to a process for the manufacture of a pharmaceutical composition comprising dapagliflozin, metformin and at least one surfactant comprising the following steps:
(i) weighing, sieving and mixing dapagliflozin,metformin and at least one surfactant, and
(ii) compressing the mixture obtained in step (i) to form a tablet
(iii) optionally, coating the resulting tablet of step (ii).

In another embodiment, the present invention relates to a process for the manufacture of a pharmaceutical composition comprising dapagliflozin, metformin and at least one surfactant comprising the following steps:
(i) weighing, sieving and mixing dapagliflozin, metformin with at least one surfactant, and
(ii) granulating the mixture obtained in step (i)
(iii) optionally, mixing the granules obtained in step (ii) with at least one pharmaceutically acceptable excipient, and
(iv) compressing the granules obtained in step (ii) or the mixture obtained in step (iii) to form a tablet
(v) optionally coating the resulting tablet of step (iv).

In another embodiment, the present invention relates to a process for the manufacture of a pharmaceutical composition comprising dapagliflozin, metformin and at least one surfactant, comprising the following steps:
(i) weighing, sieving and mixing dapagliflozin, metformin and at least one surfactant with at least one solvent, preferably wherein the solvent is free of organic solvent, and
(ii) granulating the mixture obtained in step (i)
(iii) optionally, mixing the granules obtained in step (ii) with at least one pharmaceutically acceptable excipient, and
(iv) compressing the granules obtained in step (ii) or the mixture obtained in step (iii) to form a tablet
(v) optionally coating the resulting tablet of step (iv).

In another embodiment, the present invention relates to a process for the manufacture of a pharmaceutical composition comprising dapagliflozin, metformin and at least one surfactant, comprising the following steps:
(i) weighing, sieving and mixing dapagliflozin, metformin with and at least one surfactant, and
(ii) optionally, granulating the mixture obtained in step (i)
(iii) encapsulating the mixture obtained in step (i) or the granules obtained in step (ii)

### THIRD ASPECT

In a third aspect, the present invention relates to the use of the pharmaceutical composition of the first aspect as a medicament. Preferably, for use in treating diabetes mellitus type 2, for the treatment of symptomatic chronic heart failure with reduced ejection fraction and for the treatment of chronic kidney disease.

Each of the aspects and embodiments of the present invention described herein may be combined with one or more aspects and embodiments of the present invention.

### EXAMPLES

The examples, which are incorporated and form part of the specification, merely illustrate certain embodiments of the present invention and should not be construed as limiting the invention. They are meant to serve to explain specific modes of the present invention to those skilled in the art

All examples of the invention have been carried out by using SC-3 crystalline dapagliflozin (S)-propylene glycol monohydrate ((S)-PG (SC-3)) form with a particle size volume distribution D90 of 7 microns and a particle size volume distribution D90 of 35 microns. Good stability and dissolution profiles have been obtained with both particle sizes.

### Examples 1 and 2

Wet granulated tablets according to the pharmaceutical composition of the present invention were prepared. The ingredients are shown in Table 1.

**Table 1**

| **Ingredients (mg/tablet)** | **Functionality** | **Example 1** | **Example 2** |
|---|---|---|---|
| Intragranular excipients | | | |
| Dapagliflozin | Active Ingredient | 5 | 5 |
| Sodium Lauryl Sulphate | Surfactant | 1 | 1 |
| Metformin Hydrochloride | Active Ingredient | 1000 | 1000 |
| Water | Solvent | q.s. | q.s. |

| Extragranular excipients | | | |
|---|---|---|---|
| Isomaltose | Filler | 334 | 320 |
| Sodium croscarmellose | Disintegrant | 15 | 7 |
| Colloidal silicon oxide | Glidant | 15 | 15 |
| Stearic acid | Lubricant | 50 | 72 |
| Total | | 1420 | 1420 |

| | | | |
|---|---|---|---|
| *q.s.: Quantum satis | | | |

Dapagliflozin was first mixed in water. After, sodium lauryl sulfate was added until total dissolution. Then the solution was spray-dried in metformin granules and wet-granulated in a high-shear wet-granulator. The wet-granule mass was wet-milled, then dried in a fluid-bed dryer and the dried granules were milled.

Then extragranular ingredients (isomaltose, sodium croscarmellose and colloidal silicon dioxide) were separately weighed, screened and blended with the granules. Stearic acid was added and blended with the mixture. The blend was compressed.

### Examples 3 and 4

Wet granulated tablets according to the pharmaceutical composition of the present invention were prepared. The ingredients are shown in Table 2.

**Table 2**

| **Ingredients (mg/tablet)** | **Functionality** | **Example 3** | **Example 4** |
|---|---|---|---|
| Intragranular excipients | | | |
| Dapagliflozin | Active Ingredient | 5 | 5 |
| Sodium Lauryl Sulphate | Surfactant | 1 | 1 |
| Metformin Hydrochloride | Active Ingredient | 1000 | 1000 |
| Water | Solvent | qs | qs |

| Extragranular excipients | | | |
|---|---|---|---|
| Calcium phosphate | Filler | 334 | 320 |
| Sodium starch glycolate | Disintegrant | 15 | 7 |
| Colloidal silicon oxide | Glidant | 15 | 15 |
| Stearic acid | Lubricant | 50 | 72 |
| Total | 1420 | 1420 | |

| | | | |
|---|---|---|---|
| *q.s.: Quantum satis | | | |

Dapagliflozin was first mixed in water. After, sodium lauryl sulfate was added until total dissolution. Then the solution was spray-dried in metformin granules and wet-granulated in a high-shear wet-granulator. The wet-granule mass was wet-milled, then dried in a fluid-bed dryer and the dried granules were milled.

Then extragranular ingredients (calcium phosphate, sodium starch glycolate and colloidal silicon dioxide) were separately weighed, screened and blended with the granules. Stearic acid was added and blended with the mixture. The blend was compressed.

### Examples 5 and 6

Wet granulated tablets according to the pharmaceutical composition of the present invention were prepared. The ingredients are shown in Table 3.

**Table 3**

| **Ingredients (mg/tablet)** | **Functionality** | **Example 5** | **Example 6** |
|---|---|---|---|
| Intragranular excipients | | | |
| Dapagliflozin | Active Ingredient | 5 | 5 |
| Sodium Lauryl Sulphate | Surfactant | 1 | 1 |
| Metformin Hydrochloride | Active Ingredient | 1000 | 1000 |
| Copovidone | Binder | 35.5 | 35 |
| Water | Solvent | qs | qs |

| Extragranular excipients | | | |
|---|---|---|---|
| Calcium phosphate Dibasic | Filler | 233.5 | 165 |
| Sodium starch glycolate | Disintegrant | 14 | 57 |
| Colloidal silicon dioxide | Glidant | 15 | 15 |
| Sodium stearate fumarate | Lubricant | 44 | 71 |
| Stearic acid | Lubricant | 72 | 71 |
| Total | | 1420 | 1420 |

| | | | |
|---|---|---|---|
| *q.s.: Quantum satis | | | |

Dapagliflozin was first mixed in water with copovidone. After, sodium lauryl sulfate was added until total dissolution. Then the solution was spray-dried in metformin granules and wet-granulated in a high-shear wet-granulator. The wet-granule mass was wet-milled, then dried in a fluid-bed dryer and the dried granules were milled.

Then extragranular ingredients (calcium phosphate dibasic, sodium starch glycolate, and colloidal silicon dioxide) were separately weighed, screened and blended with the granules. stearic acid and sodium stearate fumarate were added and blended with the mixture. The blend was compressed.

### Example 7 - Dissolution profiles

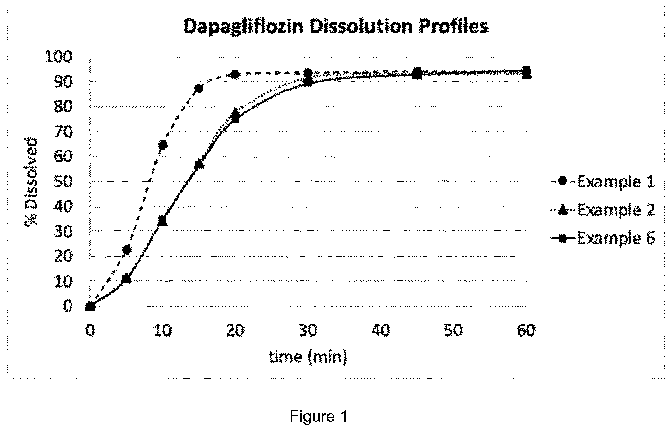

Figure 1 shows Dapagliflozin dissolution profiles of composition from Examples 1, 2 and 6.

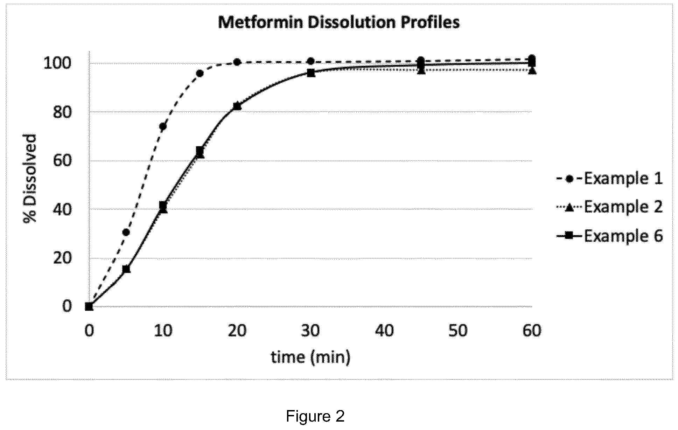

Figure 2 shows Metformin dissolution profiles of composition from Examples 1, 2 and 6.

The dissolution profiles have been carried out at pH 1.2 (NaCl/HCl) using a USP Type II apparatus, 50 rpm, 37°C in a 900 mL medium.

Tablets of examples 1 to 6, show excellent uniformity, stability, hardness, friability, disintegration and dissolution profiles. In addition, the composition developed is simple and does not require the use of complicated preparation procedures. Furthermore, the formulation of the present invention contains only a few excipients and, in particular, is free of organic solvent.

## Claims

1. A pharmaceutical composition comprising dapagliflozin, metformin and at least one surfactant.

2. The pharmaceutical composition according to claim 1 wherein the at least one surfactant is selected from polyoxyethylene sorbitan fatty acid ester, polysorbates (for example polysorbate 20, polysorbate 40, polysorbate 60, polysorbate 65, polysorbate 80, polysorbate 85, micro-encapsulated polysorbate 80 such as SEPITRAPTM 80, micro-encapsulated polyoxyl 40 hydrogenated castor oil such as SEPITRAPTM 4000), polyoxyethylene castor oil derivatives polyoxyethylene hydrogenated castor oil (for example CREMOPHOR), alkyl polyethylene oxide), copolymers of polyethylene oxide) and poly(proplylene oxide) commercially called as poloxamers or poloxamines, polyoxyethylene alkyl ethers, polyoxyethylene alkylaryl ethers, polyethylene glycol fatty acid ester, polyethylene alkyl ether, polyethylene stearate, alkylene glycol fatty acid mono ester, polyoxyethylene stearates, sucrose esters of fatty acids, PEG-8 Caprylic-Capric glycerides (DUBCARE GPE 810), saturated polyglycolized glycerides, ethoxylated hydrogenated castor oil, phosphatidyl choline, phospholipids, medium chain triglycerides, docusate sodium, lecithin, sorbitan monostearate (SPAN 860), sorbitan monopalmitate (SPAN 40), sorbitan monolaurate (SPAN 20 or SPAN 80), ascorbyl palmitate, sodium lauryl sulfate, sodium dodecyl sulfate, ammonium lauryl sulfate, benzalkonium chloride, tocopherol, tocopherol PEG succinate, and mixtures thereof.

3. The pharmaceutical composition according to claim 2 wherein the at least one surfactant is selected from sodium lauryl sulphate, polysorbates for example polysorbate 20, polysorbate 40, polysorbate 60, polysorbate 65, polysorbate 80, polysorbate 85, micro-encapsulated polysorbate 80 such as SEPITRAPTM 80, micro-encapsulated polyoxyl 40 hydrogenated castor oil such as SEPITRAPTM 4000 and mixtures thereof.

4. The pharmaceutical composition according to any of the preceding claims wherein dapagliflozin is in the form of dapagliflozin (S) propylene glycol monohydrate and metformin is in the form of hydrochloride.

5. The pharmaceutical composition according to claim 4 wherein dapagliflozin (S) propylene glycol monohydrate is in a crystalline form, preferably in the form of SC-3.

6. The pharmaceutical composition according to any of the preceding claims further comprising at least one filler, disintegrant, lubricant, glidant, solvent, binder and mixtures thereof.

7. The pharmaceutical composition according to any of the preceding claims which is free of organic solvents.

8. The pharmaceutical composition according to any of the preceding claims which is in the form of immediate release dosage form.

9. The pharmaceutical composition according to any of the previous claims which is in the form of tablet or a capsule, preferably a tablet.

10. The pharmaceutical composition according to any of the preceding claims wherein Dapagliflozin has a particle size volume distribution D90 of less than 200 microns, wherein the particle size distribution is by volume and determined by laser-diffraction spectroscopy method.

11. The pharmaceutical composition according to claim 9 wherein Dapagliflozin has a particle size volume distribution D90 of less than 100 microns, wherein the particle size distribution is by volume and determined by laser-diffraction spectroscopy method.

12. A process for the manufacture of a pharmaceutical composition according to any of the preceding claims comprising the steps of:
(i) weighing, sieving and mixing dapagliflozin, metformin and at least one surfactant, and
(ii) compressing the mixture obtained in step (i) to form a tablet
(iii) optionally, coating the resulting tablet of step (ii).

13. A process for the manufacture of a pharmaceutical composition according to any of the preceding claims comprising the steps of:
(i) weighing, sieving and mixing dapagliflozin, metformin and at least one surfactant and
(ii) granulating the mixture obtained in step (i)
(iii) optionally, mixing the granules obtained in step (ii) with at least one pharmaceutically acceptable excipient, and
(iv) compressing the granules obtained in step (ii) or the mixture obtained in step (iii) to form a tablet
(v) optionally coating the resulting tablet of step (iv).

14. The pharmaceutical composition according to any of the preceding claims for use as a medicament.

15. The pharmaceutical composition according to any of the preceding claims for use in the treatment of diabetes mellitus type 2, symptomatic chronic heart failure with reduced ejection fraction and chronic kidney disease.
